# EUROPEAN PATENT APPLICATION

(11) **EP 1 582 200 A1**
(43) Date of publication of application: **05.10.2005**
(21) Application number: 05380018.1
(22) Date of filing: 04.02.2005
(51) Int. Cl.: A61K 7/155

(54) **Cosmetic depilatory composition**

(30) Priority: 31.03.2004 ES 200400777
(71) Applicant: Martinez De San Vicente Oliveras, Luis, 08940 Cornella De Llobregat (ES)
(72) Inventor: Martinez De San Vicente Oliveras, Luis, 08940 Cornella De Llobregat (ES)
(74) Representative: SUGRANES - VERDONCES - FERREGÜELA

(57) **Abstract**

Cosmetic depilatory composition, which comprises cocoa and/or derivatives of cocoa and/or chocolate in a total amount of between 0.01% and 5% by weight. The composition is in the form of fat-soluble or hydro-soluble wax.

## Description

### Technical field of the invention

This invention is related to a cosmetic depilatory composition.

### Background of the invention

The removal of hair from the surface of the skin, or depilation, is an aesthetic concern to which much attention has been paid. Hair can be pulled out or shaved, depending which depilation technique is used.

There are numerous techniques for removing hair and the hair follicles. The techniques of electric and laser depilation are well known, although these are not usually used in the home, for the obvious reason that they require special equipment.

One of the most well-known and widely-used techniques, particularly in the home, is hair removal using hot, warm or cold wax, which due to its adhesive effect sticks strongly to the hair and allows it to be pulled out together with the hair follicle. In the past, mixtures of honey and bees' wax were used, having first been heated and then mixed with citric acid until they acquired a creamy texture. These mixtures caused irritation to the skin, which has been reduced with the development of more gentle waxes, which include in their compositions substances that soothe the skin. An example of a composition of this type is that which is disclosed in US patent 4, 832, 949.

Another example of depilatory wax is that disclosed in EP patent 0984767 B1, which presents a water-soluble depilatory wax containing a mixture of sugars and which is easily removed by rinsing with water.

EP patent 0795314 A1 also discloses a depilatory composition which contains at least one latex polymer and one resin as an adhesive, and which hardens on drying, allowing it to be applied without any kind of accessory.

One alternative to waxing is the use of depilatory creams, gels or foams, which remove hair without pulling out the follicle.

As was mentioned above, the compositions used in a wax or cream form usually contain aromatic or medicinal plant extracts such as camomile, lime blossom, aloe vera, oats and calendula, among others, to give a soothing effect to the skin where the depilatory composition is applied, thus making the depilation session more pleasant. Extracts of natural substances also tend to be used in order to reduce the unpleasant smell of some of the active ingredients in the cosmetic depilatory composition.

In the cosmetics industry, the effects of the trace element zinc (Zn) are well known for its ability to exchange with other toxic elements, which are generally coordinated with proteins. For this reason, there are numerous cosmetics which include said trace element in their composition, particularly those which are used for prevention against the effect of the sun's rays, free radicals or the ageing of the fibres of the epidermis.

The object of this invention is a cosmetic depilatory composition, with new characteristics and numerous advantages over known compositions for depilation.

### Explanation of the invention

The cosmetic depilatory composition of this invention is characterised in that it comprises cocoa and/or derivatives of cocoa and/or chocolate.

According to another characteristic of the invention, the composition contains a total amount of cocoa and/or derivatives of cocoa and/or chocolate of between 0.01% and 5% by weight.

The cosmetic composition is characterised in that it is in the form of fat-soluble or water-soluble wax.

According to another characteristic of the invention, the cocoa derivatives are chosen from the group comprised of at least cocoa oil, cocoa powder and/or hydro-glycolic extract of cocoa.

### Detailed description of the invention.

The examples below show preferred embodiments of depilatory compositions according to the invention. These examples, which must be taken as being non-limiting, are shown in order to provide people skilled in the art with a clear and complete explanation of the invention.

All the examples specify depilatory compositions comprising cocoa and/or derivatives of this and/or chocolate.

The total amount of cocoa and/or derivatives and/or of chocolate is comprised between 0.01% and 5% by weight. It has been noted that above 5 % by weight, the composition loses the consistency and/or adhesiveness needed to stay on the surface of the skin from which the hair is to be removed, consequently meaning that its depilatory properties are lost.

### Example 1.

| Fat-soluble depilatory wax with cocoa oil (per 100 g). | |
|---|---|
| Rosin | 40-55% |
| Esterified rosin with glycerine | 25-40% |
| Plasticizer | 20-30% |
| Pigments | 0.001-2% |
| Chocolate aroma | 0.1-0.5% |
| Chocolate oil (Theobroma cacao) | 0.01-0.5% |

This cosmetic composition takes the form of fat-soluble wax. It is used by heating and is applied, once warm, to the surface of the skin, allowing the hair to be pulled out by removing the wax with the aid of a cotton and/or cellulose strip.

### Example 2.

| Water-soluble depilatory wax with chocolate (per 100 g) | |
|---|---|
| Glucose | 45-60% |
| Sucrose | 20-30 % |
| Water | q.s. 100% |
| Glycerine | 2-10% |
| Citric acid | 0-5% |
| Pigments | 0.001-2 % |
| Chocolate aroma | 0.01-0.5% |
| Hydro-glycolic extract of cocoa | 0.01-0.5% |

This depilatory composition with chocolate takes the form of water-soluble wax. It is used in the same way as in example 1, but it has the advantage of allowing the removal of the wax that remains stuck to the skin, by using water once the depilation session is finished.

### Example 3.

| Water-soluble wax with cocoa powder. | |
|---|---|
| Glucose | 45-60% |
| Sucrose | 20-30% |
| Water | q.s. 100% |
| Glycerine | 2-10% |
| Citric acid | 0-5% |
| Pigments | 0.001-2 % |
| Chocolate aroma | 0.01-0.5% |
| Cocoa powder (Theobroma cacao). | 0.01-0.5% |

This composition in the form of wax has the same advantages and uses as the one described in example 2.

In all the examples, 1 to 3, the amount of cocoa and/or chocolate does not exceed 0.5% by weight. This is due to the fact that by regulation said percentage corresponds to the maximum currently allowed in this kind of cosmetic formulations.

Cocoa or its derivatives, as well as the presence of chocolate, provide the skin with an adequate amount of the trace element zinc (Zn), which is often added to cosmetic compositions due to its protective properties. Moreover, with the cocoa, the composition acquires a pleasant texture and smell.

The other ingredients of the compositions specified in examples 1 to 3 belong to the general knowledge of people skilled in the art and are usually used in the formulations of depilatory wax.

Although they are not specified, the cosmetic depilatory composition of the invention comprises, in addition to the cocoa component, other additives and ingredients which are obvious for people skilled in the art, for its correct formulation. Moreover, it is also anticipated that substances commonly used in cosmetics for their soothing properties, such as camomile, aloe vera, menthol, etc., will be added.

The cosmetic depilatory composition according to the invention is also related to all the products used for a complete depilation session, such as formulations for preparing the skin before depilation, tonics for soothing the skin once the session is finished, etc. The additives and other ingredients vary according to the type of formulation that is required, whether this be waxes, creams, tonics or oils.

The cosmetic depilatory composition in the form of fat-soluble or water-soluble waxes with cocoa and/or derivatives of cocoa and/or chocolate is applied to the skin, either cold or hot, using well-known means such as common roll-on applicators, cotton and/or cellulose and/or polyester strips which contain a fine layer of wax; using devices for heating the wax and later spreading the hot wax over the surface of the skin; etc.

The procedure for manufacturing a cosmetic depilatory composition according to the invention corresponds to the conventional procedure used to manufacture this kind of composition. The critical point in this case, is the addition of the cocoa and/or derivatives of chocolate because it must not exceed 5% by weight, the value above which the composition would lose the desired consistency for being correctly applied and thus having the desired effect.

Obviously, any modification to the depilatory composition with cocoa and/or its derivatives and/or chocolate, as well as to its method of application, are also the object of this invention.

## Claims

1. Cosmetic depilatory composition, **characterised in that** it comprises cocoa and/or derivatives of cocoa and/or chocolate.

2. Composition according to claim 1, **characterised in that** it contains a total amount of cocoa and/or derivatives of cocoa and/or chocolate of between 0.01 % and 5% by weight.

3. Composition according to any of the preceding claims, **characterised in that** it is in the form of fat-soluble or water-soluble wax.

4. Composition according to claims 1 and 2, **characterised in that** the derivatives of cocoa are chosen from the group comprised of at least cocoa oil, cocoa powder and/or hydro-glycolic extract of cocoa.
